# EUROPÄISCHE PATENTSCHRIFT

(11) **EP 2 621 537 B1**
(45) Veröffentlichungstag und Bekanntmachung des Hinweises auf die Patenterteilung: **25.02.2015**
(21) Anmeldenummer: 11764171.2
(22) Anmeldetag: 30.09.2011
(51) Int. Cl.: A61K 47/48, C07K 7/06, A61K 8/64, A61K 38/08

(54) **PENTAPEPTIDDERIVATE ZUR FÖRDERUNG DES HAARWACHSTUMS**
PENTAPEPTIDE DERIVATIVES FOR PROMOTING HAIR GROWTH
DÉRIVÉS DE PENTAPEPTIDES POUR PROMOUVOIR LA CROISSANCE DES CHEVEUX

(30) Priorität: 01.10.2010 CH 16092010
(43) Veröffentlichungstag der Anmeldung: 07.08.2013
(73) Patentinhaber: Elfetin Cosmetics AG, 4153 Reinach (CH)
(72) Erfinder: LÜTHY, Markus, CH-4144 Arlesheim (CH)
(74) Vertreter: Braun, André jr.
(86) Internationale Anmeldenummer: PCT/EP2011/067093
(87) Internationale Veröffentlichungsnummer: WO 2012/042010

(56) Entgegenhaltungen:
- EP-A1- 1 741 719
- US-A1- 2009 215 702

### SEQUENCE LISTING

<110> Elfetin Cosmetics AG
<120> Pentapeptidderivate zur Förderung des Haarwachstums
<130> P22334PC00
<140> PCT/EP2011/067093
   <141> 2011-09-30
<160> 23
<170> PatentIn version 3.5
<210> 1
   <211> 5
   <212> PRT
   <213> Artificial Sequence
<220>
   <223> Pentapeptidderivat
<220>
   <221> MOD_RES
   <222> (1)..(1)
   <223> formylation of Gly
<400> 1
   Xaa Pro Ile Gly Ser
   1               5
<210> 2
   <211> 5
   <212> PRT
   <213> Artificial Sequence
<220>
   <223> Pentapeptidderivat
<220>
   <221> MOD_RES
   <222> (1)..(1)
   <223> acetylation of Gly
<400> 2
   Xaa Pro Ile Gly Ser
   1 5
<210> 3
   <211> 5
   <212> PRT
   <213> Artificial Sequence
<220>
   <223> Pentapeptidderivat
<220>
   <221> MOD_RES
   <222> (1)..(1)
   <223> neopentanoylation of Gly
<400> 3
   Xaa Pro Ile Gly Ser
   1 5
<210> 4
   <211> 5
   <212> PRT
   <213> Artificial Sequence
<220>
   <223> Pentapeptidderivat
<220>
   <221> MOD_RES
   <222> (1)..(1)
   <223> n-hexanoylation of Gly
<400> 4
   Xaa Pro Ile Gly Ser
   1 5
<210> 5
   <211> 5
   <212> PRT
   <213> Artificial Sequence
<220>
   <223> Pentapeptidderivat
<220>
   <221> MOD_RES
   <222> (1)..(1)
   <223> n-decanoylation of Gly
<400> 5
   Xaa Pro Ile Gly Ser
   1 5
<210> 6
   <211> 5
   <212> PRT
   <213> Artificial Sequence
<220>
   <223> Pentapeptidderivat
<220>
   <221> MOD_RES
   <222> (1)..(1)
   <223> palmitoylation of Gly
<400> 6
   Xaa Pro Ile Gly Ser
   1               5
<210> 7
   <211> 5
   <212> PRT
   <213> Artificial Sequence
<220>
   <223> Pentapeptidderivat
<220>
   <221> MOD_RES
   <222> (1)..(1)
   <223> carnitinoylation of Gly
<400> 7
   Xaa Pro Ile Gly Ser
   1               5
<210> 8
   <211> 5
   <212> PRT
   <213> Artificial Sequence
<220>
   <223> Pentapeptidderivat
<220>
   <221> MOD_RES
   <222> (1)..(1)
   <223> pantothenoylation of Gly
<400> 8
   Xaa Pro Ile Gly Ser
   1               5
<210> 9
   <211> 5
   <212> PRT
   <213> Artificial Sequence
<220>
   <223> Pentapeptidderivat
<220>
   <221> MOD_RES
   <222> (1)..(1)
   <223> biotinoylation of Gly
<400> 9
   Xaa Pro Ile Gly Ser
   1               5
<210> 10
   <211> 5
   <212> PRT
   <213> Artificial Sequence
<220>
   <223> Pentapeptidderivat
<220>
   <221> MOD_RES
   <222> (1)..(1)
   <223> liponoylation of Gly
<400> 10
   Xaa Pro Ile Gly Ser
   1               5
<210> 11
   <211> 5
   <212> PRT
   <213> Artificial Sequence
<220>
   <223> Pentapeptidderivat
<220>
   <221> MOD_RES
   <222> (1)..(1)
   <223> formylation of Gly
<400> 11
   Xaa Pro Ile Gly Thr
   1               5
<210> 12
   <211> 5
   <212> PRT
   <213> Artificial Sequence
<220>
   <223> Pentapeptidderivat
<220>
   <221> MOD_RES
   <222> (1)..(1)
   <223> acetylation of Gly
<400> 12
   Xaa Pro Ile Gly Thr
   1               5
<210> 13
   <211> 5
   <212> PRT
   <213> Artificial Sequence
<220>
   <223> Pentapeptidderivat
<220>
   <221> MOD_RES
   <222> (1)..(1)
   <223> neopentanoylation of Gly
<400> 13
   Xaa Pro Ile Gly Thr
   1               5
<210> 14
   <211> 5
   <212> PRT
   <213> Artificial Sequence
<220>
   <223> Pentapeptidderivat
<220>
   <221> MOD_RES
   <222> (1)..(1)
   <223> n-hexanoylation of Gly
<400> 14
   Xaa Pro Ile Gly Thr
   1               5
<210> 15
   <211> 5
   <212> PRT
   <213> Artificial Sequence
<220>
   <223> Pentapeptidderivat
<220>
   <221> MOD_RES
   <222> (1)..(1)
   <223> n-decanoylation of Gly
<400> 15
   Xaa Pro Ile Gly Thr
   1 5
<210> 16
   <211> 5
   <212> PRT
   <213> Artificial Sequence
<220>
   <223> Pentapeptidderivat
<220>
   <221> MOD_RES
   <222> (1)..(1)
   <223> palmitoylation of Gly
<400> 16
   Xaa Pro Ile Gly Thr
   1 5
<210> 17
   <211> 5
   <212> PRT
   <213> Artificial Sequence
<220>
   <223> Pentapeptidderivat
<220>
   <221> MOD_RES
   <222> (1)..(1)
   <223> carnitinoylation of Gly
<400> 17
   Xaa Pro Ile Gly Thr
   1 5
<210> 18
   <211> 5
   <212> PRT
   <213> Artificial Sequence
<220>
   <223> Pentapeptidderivat
<220>
   <221> MOD_RES
   <222> (1)..(1)
   <223> pantothenoylation of Gly
<400> 18
   Xaa Pro Ile Gly Thr
   1 5
<210> 19
   <211> 5
   <212> PRT
   <213> Artificial Sequence
<220>
   <223> Pentapeptidderivat
<220>
   <221> MOD_RES
   <222> (1)..(1)
   <223> biotinoylation of Gly
<400> 19
   Xaa Pro Ile Gly Thr
   1               5
<210> 20
   <211> 5
   <212> PRT
   <213> Artificial Sequence
<220>
   <223> Pentapeptidderivat
<220>
   <221> MOD_RES
   <222> (1)..(1)
   <223> liponoylation of Gly
<400> 20
   Xaa Pro Ile Gly Thr
   1               5
<210> 21
   <211> 4
   <212> PRT
   <213> Artificial Sequence
<220>
   <223> Stand der Technik: EP-A-0 765 152
<220>
   <221> METAL
   <222> (4)..(4)
   <223> Cu
<400> 21
   Gly His Lys Xaa
   1
<210> 22
   <211> 6
   <212> PRT
   <213> Artificial Sequence
<220>
   <223> Stand der Technik: WO-2005/082395
<400> 22
   Arg Pro Leu Lys Pro Trp
   1 5
<210> 23
   <211> 5
   <212> PRT
   <213> Artificial Sequence
<220>
   <223> Stand der Technik: WO-2005/095441
<400> 23
   Gly Pro Ile Gly Ser
   1 5

## Beschreibung

Die Erfindung betrifft substituierte Pentapeptidverbindungen, deren Verwendung zur Herstellung kosmetischer dermatologisch wirksamer Zusammensetzungen, diese Zusammensetzungen, deren Verwendung als Haarwuchsförderer sowie ein kosmetisches Verfahren zur Förderung des Haarwachstums.

Der Mensch besitzt ungefähr 100'000 bis 150'000 Haare. Man unterscheidet drei Phasen im Haarwachstumszyklus, den jedes einzelne Haar durchläuft: die anagene, die katagene und die telogene Phase. Auf die Anagenphase (aktive Phase oder Wachstumsphase), die mehrere Jahre dauert und in deren Verlauf sich die Haare verlängern, folgt eine sehr kurze und vorübergehende Katagenphase, die einige Wochen dauert. Im Laufe dieser Phase atrophiert der Follikel, und sein Einbau in die Dermis erscheint immer höher. Die abschliessende Telogenphase, die mehrere Monate dauert, ist die Ruhephase des Follikels, während der das Haar ausfällt. Am Ende dieser Ruheperiode wird an gleicher Stelle ein neuer Follikel erzeugt, und ein neuer Zyklus beginnt.

Dieser Zyklus wiederholt sich bei gesunden Menschen in einem Intervall von 3 bis 6 Jahren mit dem Resultat, dass pro Tag zwischen 60 und 100 Haare ausfallen. Andererseits nimmt bei Menschen, bei denen dieser Zyklus gestört ist, der Anteil der Haare in der anagenen Phase ab, während der Anteil der Haare in den katagenen und telogenen Phasen zunimmt, sodass die Zahl der ausfallenden Haare abnormal ansteigt.

Haarschwund kann bei Betroffenen ernsthafte psychische Probleme auslösen bis hin zu einem Verlust des Selbstvertrauens. Deshalb ist es vordringlich, übermässigen Haarausfall am Anfang seines Entstehens zu behandeln.

Weltweit sind verschiedene Ansätze zur Behandlung des Haarausfalls und zur Förderung des Haarwachstums bei Männern und Frauen ausprobiert worden. Dabei zeigen die meisten der bisher bekannten Wirkstoffe gegen Haarverlust sowie der Nährstoffe für Haare keine ausreichend starke Wirkung; zudem haben sie häufig das Risiko von toxischen Nebeneffekten für den menschlichen Körper.

Minoxidil, der bekannteste und in den USA durch die FDA bewilligte Haarwuchsförderer für die externe Anwendung, induziert die anagene aus der telogenen Phase und hält diese fortlaufend konstant (British Journal of Dermatology, 150, 186 - 194, 2004). Allerdings zeigt Minoxidil nur eine schwache Wirkung und diese auch nur dann, wenn es ohne Unterbruch zweimal täglich angewendet wird und jeweils für 3 bis 4 Stunden auf der Kopfhaut verbleibt. Zur unzureichenden Wirkung kommen Nebeneffekte, wie Juckreiz oder Hautreizung, hinzu.

Auch oral anwendbare Wirkstoffe, wie Finasterid, welche die 5-alpha-Reduktase hemmen, werden als durch die FDA bewilligte Haarwuchsstimulatoren vermarktet. Diese sind jedoch teuer und zeigen Nebeneffekte, wie Impotenz oder Teratogenität.

Zu den natürlichen Wirkstoffen gehören die Inhaltsstoffe der Sophorawurzel, welche die Haarwachstumsfaktoren IGF-1 und KGF erhöhen; die Procyanidine, welche die Haardicke und die Haardichte steigern; Vitamin A, welches die Neubildung von Haarzellen anregt; das den Haarwuchs anregende Vitamin D; Arginin für das Wachstum der Haarzellen; der Creatinethylester, der die Energie für die Haarzellen liefert; und L-Carnitin für den verbesserten Nährstofftransport. Allen diesen Stoffen fehlt aber eine ausreichend starke Wirkung gegen Alopecia.

Zu den peptidischen Wirkstoffen, welche das Haarwachstum positiv beeinflussen, gehört das körpereigene und auch als synthetisches Produkt vermarktete Gly-His-Lys-Cu (EP-A-0 765 152), dessen enzymatische Stabilität jedoch sehr gering ist. Ebenso gehört das Hexapeptid Arg-Pro-Leu-Lys-Pro-Trp (WO-2005/082395) dazu, welches jedoch bisher keine Marktreife erlangt hat.

Neuere Forschungsergebnisse zur Förderung des Haarwuchses sind in Biol. Pharm. Bull., 28(3), 485 - 489, (2005) und in WO-2005/095441 publiziert. Beschrieben wird darin das wasserlösliche Pentapeptid Gly-Pro-Ile-Gly-Ser, welches das Wachstum der Haarbulbuskeratinozyten begünstigt und dadurch das Neuwachstum der Haare in telogenen Mäusen anregt. Dieses Peptid erfüllt jedoch die Voraussetzungen für eine genügende Hautpermeation nicht.

Es ist Aufgabe der Erfindung, kosmetische Wirkstoffe zur Verfügung zu stellen, welche das Haarwachstum ausreichend stark fördern, allenfalls geringe physiologisch nachteilige Nebeneffekte zeigen sowie genügend hautdurchlässig und ausreichend biologisch stabil sind.

Ein erster Aspekt der Erfindung löst die Aufgabe durch Verbindungen der Formel

R¹-Gly-Pro-Ile-Gly-A₁ (I),

worin
A₁ Ser oder Thr ist und
R¹ -C(=O)-H, -C(=O)-C₁-C₂₄-Alkyl, -C(=O)-CH₂CH(OH)CH₂N⁺(CH₃)₃ (Carnitinoyl), 1,2-Dithiolan-3-pentanoyl (Liponoyl), -C(=O)-(CH₂)₂NHC(=O)CH(OH)C(CH₃)₂CH₂OH (Pantothenoyl) oder 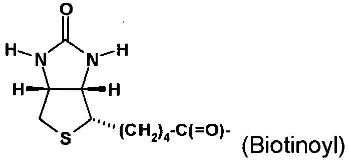 bedeutet.

Verbindungen I können in Racemat-Form oder in enantiomerenreiner Form sowie in freier Form oder in Salzform, vorzugsweise in dermatologisch verträglicher Salzform, vorliegen, z. B. als Salz mit einer Base, wie Natrium-, Kalium-, Calcium- oder Ammoniumhydroxid, oder als innere Salze (Zwitterionen; Betaine).

Die Substitution des Basispentapeptids mit einer der lipophilen Gruppen R¹ zu den Verbindungen I erhöht nicht nur den log P-Wert (Mass der Lipophilie / Hautdurchlässigkeit), sondern überraschenderweise auch erheblich die Stabilität der Verbindungen I gegen den enzymatischen Abbau durch Peptidasen und führt dadurch zu einer länger anhaltenden Stimulation des Haarwachstums, verglichen mit den entsprechenden Werten für das unsubstituierte Basispeptid. Ebenso überraschend zeigt sich ein stärkeres Haarbulbuskeratinozyten-Wachstum.

Unter "C₁-C₂₄-Alkyl" sind geradkettige oder verzweigte gesättigte Kohlenwasserstoffreste mit 1 bis und mit 24 Kohlenstoffatomen zu verstehen, z. B. Methyl, Ethyl, n-Propyl, n-Butyl, n-Pentyl, n-Hexyl, n-Heptyl, n-Octyl, n-Nonyl, n-Undecyl, n-Dodecyl, n-Tridecyl, n-Hexadecyl, n-Heptadecyl, n-Octadecyl, n-Nonadecyl, i-Propyl, tert.-Butyl, i-Butyl, sec.-Butyl oder i-Pentyl.

Die vorstehend und nachfolgend verwendeten Dreibuchstabenkürzel für die Aminosäure-Bausteine haben die folgenden Bedeutungen: Ser = Serin, Thr = Threonin, Gly = Glycin, Pro = Prolin, Ile = Isoleucin.

In einer bevorzugten Ausführungsform der Erfindung sind die Verbindungen I enantiomerenrein, ausgehend von den natürlichen Aminosäuren. In einer anderen bevorzugten Ausführungsform ist A₁ in den Verbindungen I Ser. Bevorzugte spezifische Verbindungen I sind in Tabelle 1 aufgeführt.

**Tabelle 1**

| **Nr.** | **R¹** | **A₁** | **Bemerkungen** |
|---|---|---|---|
| 1 | -C(=O)-H | Ser | |
| 2 | -C(=O)-CH₃ | Ser | |
| 3 | -C(=O)-C(CH₃)₃ | Ser | |
| 4 | -C(=O)-(CH₂)₄CH₃ | Ser | |
| 5 | -C(=O)-(CH₂)₈CH₃ | Ser | |
| 6 | -C(=O)-(CH₂)₁₄CH₃ | Ser | |
| 7 | -C(=O)-CH₂CH(OH)CH₂N⁺(CH₃)₃ | Ser | als inneres Salz; aus L-Carnitin |
| 8 | -C(=O)-(CH₂)₂NHC(=O)CH(OH)C(CH₃)₂CH₂OH | Ser | aus (R)-N-(2,4-Dihydroxy-3,3-dimethyl-1-oxo-butyl)-beta-alanin |
| 9 | 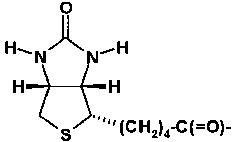 | Ser | aus (3aS,4S,6aR)-Biotin; Massenspektrum: 654.50 = (M-H)⁻ |
| 10 | 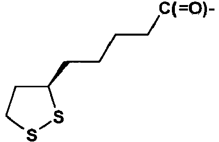 | Ser | aus (R)-(+)-5-(1,2-Dithiolan-3-yl)-pentansäure |
| 11 | -C(=O)-H | Thr | |
| 12 | -C(=O)-CH₃ | Thr | |
| 13 | -C(=O)-C(CH₃)₃ | Thr | |
| 14 | -C(=O)-(CH₂)₄CH₃ | Thr | |
| 15 | -C(=O)-(CH₂)₈CH₃ | Thr | |
| 16 | -C(=O)-(CH₂)₁₄CH₃ | Thr | |
| 17 | -C(=O)-CH₂CH(OH)CH₂N⁺(CH₃)₃ | Thr | als inneres Salz; aus L-Carnitin |
| 18 | -C(=O)-(CH₂)₂NHC(=O)CH(OH)C(CH₃)₂CH₂OH | Thr | aus (*R*)-N-(2,4-Dihydroxy-3,3-dimethyl-1-oxo-butyl)-beta-alanin |
| 19 | 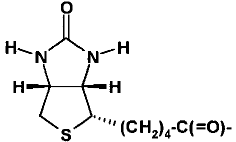 | Thr | aus (3a*S*,4*S*,6a*R*)-Biotin |
| 20 | 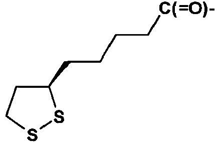 | Thr | aus (*R*)-(+)-5-(1,2-Dithiolan-3-yl)-pentansäure |

In einer besonders bevorzugten Ausführungsform der Erfindung sind die Verbindungen I ausgewählt aus der Gruppe, bestehend aus den Verbindungen, in freier Form oder in Salzform, (i) Palmitoyl-Gly-Pro-Ile-Gly-Ser-OH, (ii) Carnitinoyl-Gly-Pro-Ile-Gly-Ser-OH, (iii) 1,2-Di-thiolan-3-pentanoyl-Gly-Pro-Ile-Gly-Ser-OH, (iv) Pantothenoyl-Gly-Pro-Ile-Gly-Ser-OH und (v) 5-[(3aS,4S,6a*R*)-2-Oxo-hexahydro-1*H*-thieno[3,4-*d*]imidazol-4-yl]pentanoyl-Gly-Pro-Ile-Gly-Ser-OH, vorzugsweise aus denjenigen dieser Verbindungen, die eine -Ser-O⁻ -Teilstruktur aufweisen, wie, vorzugsweise, Carnitinoyl-Gly-Pro-Ile-Gly-Ser-O⁻.

Die Verbindungen I können nach an sich bekannten Verfahren (z. B. gemäss den allgemeinen Vorschriften in M. Bodanszky, "The Practice of Peptide Synthesis", Springer, 2nd Edition, 1994) hergestellt werden, z. B. indem man eine Aminosäure A₁, deren Hydroxy- und alpha-Amino-Gruppen geschützt sind, mittels ihrer Carboxylfunktion an eine entsprechend funktionalisierte Festphase kuppelt, sodann die alpha-Aminoschutzgruppe (z. B. eine tert.-Butyloxycarbonyl-Gruppe) abspaltet, anschliessend das Peptid stufenweise mit den in der Peptidsynthese üblichen Reagenzien aufbaut, bis das gewünschte Pentapeptid vollständig synthetisiert ist, danach die freigesetzte Aminogruppe in der Position P1 des Peptids in eine R¹-N(H)-Gruppe überführt, dann die erhaltene Verbindung von der Festphase abkuppelt, die Hydroxyschutzgruppe in der Position P5 des Peptids (z. B. eine Benzyloxycarbonyl- oder tert.-Butyloxycarbonyl-Gruppe) abspaltet und die erhaltene Verbindung I chromatographisch und / oder durch Umkristallisation reinigt.

Die Verbindungen I werden vorzugsweise als Wirkstoff in kosmetischen dermatologisch wirksamen Zusammensetzungen eingesetzt, bevorzugt in einer Konzentration zwischen 0.5 und 50000, insbesondere zwischen 0.5 und 5000, vor allem zwischen 5 und 50000, bevorzugt zwischen 1 und 500, vorzugsweise zwischen 50 und 500, ppm (w/w).

Ein weiterer Aspekt der Erfindung ist die Verwendung wenigstens einer der Verbindungen I als Wirkstoff für die Herstellung von kosmetischen dermatologisch wirksamen, vorzugsweise topisch anwendbaren, Zusammensetzungen, vorzugsweise zur Förderung des Haarwachstums.

Ein weiterer Aspekt der Erfindung ist auf kosmetische dermatologisch wirksame Zusammensetzungen gerichtet, enthaltend vorzugsweise zwischen 0.5 und 50000, insbesondere zwischen 0.5 und 5000, vor allem zwischen 5 und 50000, bevorzugt zwischen 1 und 500, vorzugsweise zwischen 50 und 500, ppm (w/w) wenigstens einer der Verbindungen I als Wirkstoff.

In den erfindungsgemässen Zusammensetzungen können die Verbindungen I vorzugsweise in Form einer Lösung, Dispersion oder Emulsion, eingekapselt in Trägersubstanzen, vorzugsweise in Makro-, Mikro- oder Nanokapseln, Liposomen oder Chylomikronen, eingeschlossen in Makro-, Mikro- oder Nanoteilchen oder Mikroschwämmen oder absorbiert auf pulverförmigen organischen Polymeren oder mineralischen Trägern, vorzugsweise Talk oder Bentonit, eingesetzt werden.

Die Verbindungen I können in den Zusammensetzungen in jeder dermatologisch geeigneten galenischen Form verwendet werden, vorzugsweise in Form von Cremen, Lotionen, Salben, Shampoos, Gelen, Pudern, Sprays, Milchprodukten, Pflastern oder Körperölen, vorzugsweise in Form von Wasser-in-Öl- oder Öl-in-Wasser-Emulsionen oder gelierenden und viskosen, spannungsaktiven und emulgierenden Polymeren.

Vorzugsweise werden die erfindungsgemässen Zusammensetzungen als kosmetisch wirksame, vorzugsweise topisch anwendbare, Mittel, bevorzugt als Haarwuchsförderer, verwendet.

In einer bevorzugten Ausführungsform enthält die erfindungsgemässe Zusammensetzung wenigstens einen zusätzlichen kosmetischen (Haut-)pflegewirkstoff, z. B. ein Extraktionslipid, ein Syntheselipid, ein gelierendes und viskoses, spannungsaktives und emulgierendes Polymer, ein wasser- oder fettlösliches Wirkprinzip, einen Pflanzenextrakt, einen Gewebeextrakt, einen Meeresextrakt, ein Sonnenschutzmittel, ein Antioxidans, ein Feuchthaltemittel, ein Barrieremittel oder einen die Haut revitalisierenden Wirkstoff, besonders bevorzugt wenigstens einen zusätzlichen Hautpflegewirkstoff ausgewählt aus der Gruppe der Haarwuchsförderer, vorzugsweise aus Biotin, Carnitin, Pantothensäure, Koffein, Minoxidil, Carnosin, Taurin, den Inhaltsstoffen der Sophorawurzel, den Procyanidinen, Vitamin A, Vitamin D und Arginin.

Die erfindungsgemässen Zusammensetzungen enthalten vorzugsweise auch einen dermatologisch annehmbaren Träger, also einen Träger, der zur dermatologischen, vorzugsweise topischen, Anwendung auf die Haut geeignet ist, vorzugsweise gute ästhetische Eigenschaften hat, mit den Verbindungen I und vorzugsweise auch den anderen Komponenten verträglich ist und keine Sicherheits- oder Toxizitätsnachteile aufweist.

Solch ein Träger kann in vielen unterschiedlichen Formen vorliegen. Beispielsweise sind Emulsionsträger, einschliesslich Öl-in-Wasser-, Wasser-in-Öl-, Wasser-in-Öl-in-Wasser- und Öl-in-Wasser-in-Silikon-Emulsionen, geeignet, z. B.
A) Wasser-in-Silikon-Emulsionen, enthaltend eine kontinuierliche Silikonphase und eine darin dispergierte wässrige Phase; oder
B) Öl-in-Wasser-Emulsionen mit einer kontinuierlichen wässrigen Phase und einer darin dispergierten hydrophoben, wasserunlöslichen Phase ("Ölphase"). Beispiele für geeignete Öl-in-Wasser-Emulsionsträger sind in US-A-5,073,371 und US-A-5,073,372 beschrieben.

Die jeweils einzusetzenden Mengen an kosmetischen und / oder dermatologischen Hilfs- und Zusatzstoffen sowie an Parfüm können in Abhängigkeit von der Art des jeweiligen Produkts vom Fachmann durch einfaches Ausprobieren leicht ermittelt werden.

Ein weiterer Aspekt der Erfindung ist auf ein kosmetisches Verfahren zur Förderung des Haarwachstums gerichtet, bei dem wenigstens eine erfindungsgemässe Verbindung oder Zusammensetzung auf das Haar, vorzugsweise den Haarboden, aufgetragen wird, wobei dieser Auftrag einmalig oder mehrfach, z. B. einmal täglich, mehrmals täglich, einmal an jedem zweiten Tag oder in anderen geeigneten Zeitabständen, während einer bestimmten Zeitspanne oder auf Dauer, erfolgen kann.

Mittels der folgenden Beispiele wird die Erfindung anhand spezifischer und nicht beschränkend auszulegender Ausführungsformen erläutert ("Gew-%" steht für Gewichtsprozent).

### Beispiel 1: Haarschaum- und Duschmittel

Ein Haarschaum- und Duschmittel wird in Analogie zu bekannten Methoden gemäss folgendem Rezept hergestellt:

| **Phase** | **Ingredienzien** | **INCI-Name** | **Gew-%** |
|---|---|---|---|
| A | Destilliertes Wasser | Aqua | 26.00 |
| | Carbopol AQUA SF-1 Polymer | Acrylates Copolymer | 7.50 |
| | Texapon NSO-BZ | Sodium Laureth Sulfate | 40.00 |
| B | Miranol Ultra C 32 | Sodium Cocoamphoacetate | 5.00 |
| | Hostapon CLG | Sodium Lauroyl Glutamate | 4.50 |
| C | Jaguar C 162 (Premix 2 %) | Hydroxypropyl Guar Hydroxypropyltrimonium Chloride | 10.00 |
| | Pentapeptidderivat Nr. 9 aus Tabelle 1 | - | 0.0025 |
| | Destilliertes Wasser | Aqua | 0.7475 |
| | Glycerin | Glycerin | 1.75 |
| | Euxyl K 300 | Phenoxyethanol (and) Methylparaben (and) Ethylparaben (and) Propylparaben (and) Butylparaben (and) Isobutylparaben | 0.80 |
| | Lime Parfüm | Fragrance | 0.50 |
| | FD & C Yellow No. 5 | Cl 19140 | q. s. |
| | Frescolat Plus | Menthyl Lactate (and) Menthol | 0.20 |
| D | Dehyton AB-30 | Coco-Betaine | 2.00 |
| | Rewoderm LI S 80 | PEG-200 Hydrogenated Glyceryl Palmate (and) PEG-7 Glyceryl Cocoate | 1.00 |
| E | Zitronensäure | Citric Acid | q. s. |

### Beispiel 2: Haar- und Kopfhautmittel

Ein Haar- und Kopfhautmittel wird in Analogie zu bekannten Methoden gemäss folgendem Rezept hergestellt:

| **Phase** | **Ingredienzien** | **INCI-Name** | **Gew-%** |
|---|---|---|---|
| A | Destilliertes Wasser | Aqua | 63.3875 |
| | Ethanol DEB 96 % | Alcohol denat. | 30.00 |
| | PVP/VA Copolymer | PVP/VA Copolymer | 2.50 |
| | Euxyl K 300 | Phenoxyethanol (and) Methylparaben (and) Ethylparaben (and) Propylparaben (and) Butylparaben (and) Isobutylparaben | 0.80 |
| B | PROTACHEM HCO-40 | PEG-40 Hydrogenated Castor Oil | 0.50 |
| | Parfüm | Fragrance | 0.10 |
| C | Triethanolamin 99 % | Triethanolamine | 0.01 |
| D | FD & C Yellow No. 5 (Lösung 0.5 %) | Cl 19140, Aqua | 0.10 |
| | FD & C Blue No. 1 (Lösung 0.5 %) | Cl 42090, Aqua | 0.10 |
| | Pentapeptidderivat Nr. 9 aus Tabelle 1 | - | 0.0025 |
| | Destilliertes Wasser | Aqua | 0.75 |
| | Glycerin | Glycerin | 1.75 |

### Beispiel 3: Lotion

Eine Lotion wird in Analogie zu bekannten Methoden gemäss folgendem Rezept hergestellt:

| **Phase** | **Ingredienzien** | **INCI-Name** | **Gew-%** |
|---|---|---|---|
| A | Pationic 138C | Sodium Lauroyl Lactylate | 0.34 |
| | Cetylalkohol | Cetyl Alcohol | 2.00 |
| | Tegin 4100 | Glyceryl Stearate | 2.00 |
| | Tegosoft TN | C12-15 Alkyl Benzoate | 7.00 |
| | Tegosoft CT | Caprylic / Capric Triglycerides | 7.00 |
| B | Destilliertes Wasser | Aqua | 76.455 |
| | Propylenglycol | Propylene Glycol | 5.00 |
| | Pentapeptidderivat Nr. 9 aus Tabelle 1 | - | 0.005 |
| | Keltrol RD | Xanthan Gum | 0.20 |
| C | Säure oder Base für die Einstellung von pH 5.0 bis 5.5 | - | q. s. |

Die Phasen A und B separat auf 75°C erwärmen, die Phase A unter Rühren zu der Phase B geben, das Gemisch homogenisieren und auf Raumtemperatur abkühlen lassen und dann den pH-Wert mittels der Phase C auf 5.0 bis 5.5 einstellen.

### Beispiel 4: Creme

Eine Creme wird in Analogie zu bekannten Methoden gemäss folgendem Rezept hergestellt:

| **Phase** | **Ingredienzien** | **INCI-Name** | **Gew-%** |
|---|---|---|---|
| A | Imwitor 372P | Glyceryl Stearate Citrate | 2.00 |
| | Cutina GMS | Glyceryl Stearate | 3.00 |
| | Sympatens-O/4200 | Sorbitan Laurate (and) Polyglyceryl-10 Laurate | 1.00 |
| | Mandelöl | Prunus Amygdalus Dulcis | 2.50 |
| | Tegosoft TN | C12-15 Alkyl Benzoate | 7.00 |
| | Cetiol OE | Dicaprylyl Ether | 5.00 |
| | Tegosoft DC | Decyl Cocoate | 3.00 |
| | Butylhydroxytoluol | BHT | 0.05 |
| | Euxyl PE 9010 | Phenoxyethanol (and) Ethylhexylglycerin | 1.00 |
| | Dow Corning 345 | Cyclopentasiloxane (and) Cyclohexasiloxane | 2.00 |
| B | Keltrol RD | Xanthan Gum | 0.30 |
| | Glycerin | Glycerin | 2.00 |
| | Destilliertes Wasser | Aqua | 68.345 |
| C | Pentapeptidderivat Nr. 9 aus Tabelle 1 | - | 0.005 |
| | Destilliertes Wasser | Aqua | 0.75 |
| | Glycerin | Glycerin | 1.75 |
| D | Rapithix A-60 | Sodium Polyacrylate (and) Hydrogenated Polydecene (and) Trideceth-6 | 0.30 |
| E | Säure oder Base für die Einstellung von pH 5 bis 6 | | q. s. |

### Beispiel 5: Bestimmung der Haarbulbuskeratinozyten-Proliferation

In dem in Biol. Pharm. Bull., 28(3), 485 - 489, (2005) beschriebenen Test bewirken Verbindungen I im Vergleich zu Gly-Pro-Ile-Gly-Ser ein ausgeprägteres Keratinozytenwachstum. So ergibt sich z. B. für das Pentapeptidderivat Nr. 9 aus Tabelle 1 in diesem Test ein um bis zu 53 % verbesserter Wert, verglichen mit dem entsprechenden Wert für Gly-Pro-Ile-Gly-Ser.

## Patentansprüche

1. Eine Verbindung der Formel
R¹-Gly-Pro-Ile-Gly-A₁ (I),
worin
A₁ Ser oder Thr ist und
R¹ -C(=O)-H, -C(=O)-C₁-C₂₄-Alkyl, -C(=O)-CH₂CH(OH)CH₂N⁺(CH₃)₃ (Carnitinoyl), 1,2-Dithiolan-3-pentanoyl (Liponoyl), -C(=O)-(CH₂)₂NHC(=O)CH(OH)C(CH₃)₂CH₂OH (Pantothenoyl) oder 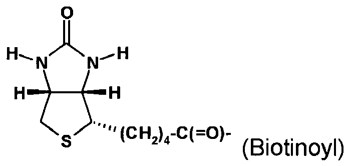 bedeutet, in freier Form oder in Salzform, vorzugsweise in dermatologisch verträglicher Salzform.

2. Eine Verbindung gemäss Anspruch 1 der Formel I in Racemat-Form oder in enantiomerenreiner Form.

3. Eine Verbindung gemäss Anspruch 1 oder Anspruch 2 der Formel I, worin A₁ Ser ist.

4. Eine Verbindung gemäss einem der Ansprüche 1 bis 3 der Formel I, ausgewählt aus der Gruppe, bestehend aus den Verbindungen, in freier Form oder in Salzform, (i) Palmitoyl-Gly-Pro-Ile-Gly-Ser-OH, (ii) Carnitinoyl-Gly-Pro-Ile-Gly-Ser-OH, (iii) 1,2-Dithiolan-3-pentanoyl-Gly-Pro-Ile-Gly-Ser-OH, (iv) Pantothenoyl-Gly-Pro-Ile-Gly-Ser-OH und (v) 5-[(3aS,4S,6aR)-2-Oxo-hexahydro-1H-thieno[3,4-d]imidazol-4-yl]pentanoyl-Gly-Pro-Ile-Gly-Ser-OH, vorzugsweise aus denjenigen dieser Verbindungen, die eine -Ser-O⁻-Teilstruktur aufweisen, wie, vorzugsweise, Carnitinoyl-Gly-Pro-Ile-Gly-Ser-O⁻.

5. Verwendung wenigstens einer Verbindung wie in einem der Ansprüche 1 bis 4 definiert der Formel I als Wirkstoff für die Herstellung einer kosmetischen dermatologisch wirksamen, vorzugsweise topisch anwendbaren, Zusammensetzung.

6. Verwendung gemäss Anspruch 5 zur Herstellung einer Zusammensetzung zur Förderung des Haarwachstums.

7. Eine kosmetische dermatologisch wirksame Zusammensetzung, enthaltend wenigstens eine Verbindung wie in einem der Ansprüche 1 bis 4 definiert der Formel I als Wirkstoff.

8. Eine Zusammensetzung gemäss Anspruch 7, enthaltend zwischen 0.5 und 50000, insbesondere zwischen 0.5 und 5000, vor allem zwischen 5 und 50000, bevorzugt zwischen 1 und 500, vorzugsweise zwischen 50 und 500, ppm (w/w) Wirkstoff.

9. Eine Zusammensetzung gemäss Anspruch 7 oder Anspruch 8, enthaltend wenigstens einen zusätzlichen Hautpflegewirkstoff ausgewählt aus der Gruppe der Haarwuchsförderer, vorzugsweise aus Biotin, Carnitin, Pantothensäure, Koffein, Minoxidil, Carnosin, Taurin, den Inhaltsstoffen der Sophorawurzel, den Procyanidinen, Vitamin A, Vitamin D und Arginin.

10. Eine Zusammensetzung gemäss einem der Ansprüche 7 bis 9, enthaltend den Wirkstoff in Form einer Lösung, Dispersion oder Emulsion, eingekapselt in Trägersubstanzen, vorzugsweise in Makro-, Mikro- oder Nanokapseln, Liposomen oder Chylomikronen, eingeschlossen in Makro-, Mikro- oder Nanoteilchen oder Mikroschwämmen oder absorbiert auf pulverförmigen organischen Polymeren oder mineralischen Trägern, vorzugsweise Talk oder Bentonit.

11. Eine Zusammensetzung gemäss einem der Ansprüche 7 bis 10 in Form einer Creme, Lotion oder Salbe oder eines Shampoos, Gels, Puders, Sprays, Milchprodukts, Pflasters oder Körperöls, vorzugsweise in Form einer Wasser-in-Öl- oder Öl-in-Wasser-Emulsion oder eines gelierenden und viskosen, spannungsaktiven und emulgierenden Polymers.

12. Verwendung einer Zusammensetzung wie in einem der Ansprüche 7 bis 11 definiert als kosmetisch wirksames, vorzugsweise topisch anwendbares, Mittel, bevorzugt als Haarwuchsförderer.

13. Ein kosmetisches Verfahren zur Förderung des Haarwachstums, **dadurch gekennzeichnet, dass** wenigstens eine Verbindung wie in einem der Ansprüche 1 bis 4 definiert der Formel I oder eine Zusammensetzung wie in einem der Ansprüche 7 bis 11 definiert auf das Haar, vorzugsweise den Haarboden, aufgetragen wird.

## Claims

1. A compound of formula
R¹-Gly-Pro-Ile-Gly-A.| (I),
where
A₁ stands for Ser or Thr and
R¹ denotes -C(=O)-H, -C(=O)-C₁-C₂₄ alkyl, -C(=O)-CH₂CH(OH)CH₂N⁺(CH₃)₃ (carnitinoyl), 1,2-Dithiolane-3-pentanoyl (liponoyl), -C(=O)-(CH₂)₂NHC(=O)CH(OH)C(CH₃)₂CH₂OH (pantothenoyl)
or 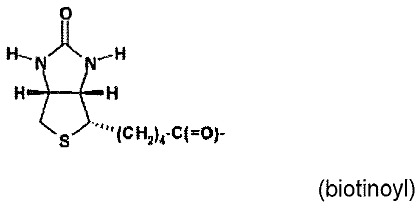
in free form or as in salt form, preferably in a dermatologically tolerated salt form.

2. The compound according to Claim 1 of formula I in racemate or enantiopure form.

3. The compound according to Claim 1 or 2 of formula I, where A₁ stands for Ser.

4. The compound according to any one of the Claims 1 to 3 of formula selected from the group of the compounds, in free form or in salt form, (i) palmitoyl-Gly-Pro-Ile-Gly-Ser-OH; (ii) carnitinoyl-Gly-Pro-Ile-Gly-Ser-OH; (iii) 1,2-dithiolane-3-pentanoyl-Gly-Pro-Ile-Gly-Ser-OH; (iv) pantothenoyl-Gly-Pro-Ile-Gly-Ser-OH; and (v) 5-[(3a*S*,4*S*,6a*R*)-2-oxo-hexahydro-1*H*-thieno[3,4-d]imidazol-4-yl]pentanoyl-Gly-Pro-Ile-Gly-Ser -OH, preferably from those of said compounds having a -Ser-O⁻ partial structure such as, for example, carnitinoyl-Gly-Pro-Ile-Gly-Ser-O⁻.

5. A use of at least one compound as defined in any one of the Claims 1 to 4 of formula I as active substance for the production of a cosmetic dermatologically active, preferably topically applied, composition.

6. The use according to Claim 5 in the production of a composition for promoting hair growth.

7. A cosmetic dermatologically active composition containing at least one compound as defined in Claims 1 to 4 of formula I as active substance.

8. A composition according to Claim 7, containing between 0.5 and 50000, particularly between 0.5 and 5000, especially between 5 and 50000, preferred between 1 and 500, preferably between 50 and 500, ppm (w/w) active substance.

9. The composition according to Claim 7 or 8, containing at least one further skin care agent selected from the group of hair growth promoters, preferably biotine, carnitine, pantothenoic acid, caffeine, minoxidil, carnosine, taurine, the ingredients of the Sophora root, procyanidins, vitamin A, vitamin D and arginine.

10. The composition according to any one of the Claims 7 to 9, containing the active substance in form of a solution, dispersion or emulsion, encapsulated in carrier substances, preferably in macro-, micro- or nano-capsules, liposomes or chylomicrons, enclosed in macro-, micro- or nano-particles or micro-sponges or absorbed on organic polymers in powder form or mineral carriers, preferably talcum or bentonite.

11. The composition according to any one of the Claims 7 to 10 in form of a cream, lotion or ointment or a shampoo, gel, powder, spray, milk product, patch or body oil, preferably in form of a water-in-oil or oil-in-water emulsion or a gelatinizing and viscous tensioactive and emulsifying polymer.

12. A use of a composition according to any one of the Claims 7 to 11 defined as cosmetically active, preferably topically applicable, agent, preferred as hair growth promoter.

13. A cosmetic method for promoting hair growth, **characterized in that** at least one compound as defined in any one of the Claims 1 to 4 of formula I, or a composition as defined in any one of the Claims 7 to 11 is applied to the hair, preferably the scalp.

## Revendications

1. Composé de la formule
R¹-Gly-Pro-Ile-Gly-A₁ (I),
où
A₁ est Ser ou Thr et
R¹ représente -C(=O)-H,-C(=O)-(C₁-C₂₄)alkyle, -C(=O)-CH₂CH(OH)CH₂N⁺(CH₃)₃ (carnitinoyle), 1,2-dithiolan-3-pentanoyl (liponoyle), -C(=O)-(CH₂)₂NHC(=O)CH(OH)C(CH₃)₂CH₂OH (pantothénoyle)
ou 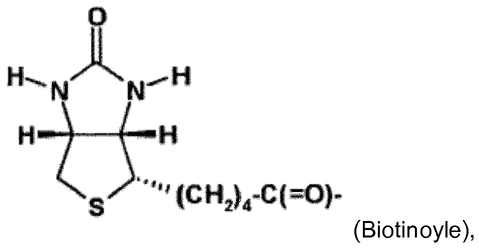
sous forme libre ou sous forme de sel, de préférence sous forme de sel dermatologiquement compatible.

2. Composé de la formule I selon la revendication 1, ledit composé se présentant sous forme racémique ou sous forme énantiomériquement pure.

3. Composé de formule la I selon la revendication 1 ou la revendication 2, où A₁ est Ser.

4. Composé de la formule I selon l'une des revendications 1 à 3, ledit composé étant choisi dans le groupe constitué par les composés, sous forme libre ou sous forme de sel, (i) palmitoyl-Gly-Pro-Ile-Gly-Ser-OH, (ii) carnitinoyl-Gly-Pro-Ile-Gly-Ser-OH, (iii) 1,2-dithiolan-3-pentanoyl-Gly-Pro-Ile-Gly-Ser-OH, (iv) pantothénoyl-Gly-Pro-Ile-Gly-Ser-OH, (v) 5-[(3a*S*,4*S*,6a*R*)-2-oxo-hexahydro-1*H*-thiéno[3,4-*d*]imidazol-4-yl]pentanoyl-Gly-Pro-Ile-Gly-Ser-OH, de préférence sélectionné parmi ceux des composés qui ont une structure partielle -Ser-O⁻-, comme de préférence le carnitinoyl-Gly-Pro-Ile-Gly-Ser-O-.

5. Utilisation d'au moins un composé de la formule I selon l'une des revendications 1 à 4 comme principe actif pour la préparation d'une composition cosmétique dermatologiquement efficace, de préférence topiquement applicable.

6. Utilisation selon la revendication 5 pour la préparation d'une composition destinée à favoriser la pousse des cheveux.

7. Composition cosmétique dermatologiquement efficace, contenant au moins un composé de la formule I selon l'une des revendications 1 à 4 comme principe actif.

8. Composition selon la revendication 7, contenant entre 0,5 et 50000, notamment entre 0,5 et 5000, surtout entre 5 à 50000, de préférence entre 1 et 500, de préférence entre 50 et 500, ppm (p/p) de principe actif.

9. Composition selon la revendication 7 ou la revendication 8, contenant au moins un principe actif supplémentaire de soin de la peau choisi dans le groupe des promoteurs de la croissance des cheveux, de préférence parmi la biotine, la carnitine, l'acide pantothénique, la caféine, le minoxidil, la carnosine, la taurine, les substances de la racine de sophora. les procyanidines, la vitamine A, la vitamine D et l'arginine.

10. Composition selon l'une des revendications 7 à 9, contenant le principe actif sous la forme d'une solution, d'une dispersion ou d'une émulsion, encapsulée dans des substances support, de préférence dans des macro-, micro- ou nanocapsules, des liposomes ou des chylomicrons, enfermée dans des macro-, micro- ou nanoparticules ou des micro-éponges ou absorbée sur des polymères organiques ou des supports minéraux en poudre, de préférence du talc ou de la bentonite.

11. Composition selon l'une des revendications 7 à 10 sous la forme d'une crème, d'une lotion ou d'une pommade ou d'un shampooing, d'un gel, d'une poudre, d'un spray, d'un lait, d'un patch ou d'une huile corporelle, de préférence sous la forme d'une émulsion eau-dans-huile ou huile-dans-eau ou d'un polymère gélifiant et visqueux, tensioactif et émulsifiant.

12. Utilisation d'une composition selon l'une des revendications 7 à 11 comme agent cosmétiquement efficace, de préférence topiquement applicable, de préférence comme promoteur de la croissance des cheveux.

13. Procédé cosmétique destiné à favoriser la croissance des cheveux, **caractérisé en ce qu'**au moins un composé de la formule I selon l'une des revendications 1 à 4 ou une composition selon l'une des revendications 7 à 11, est appliqué sur les cheveux, de préférence le cuir chevelu.
